# EUROPEAN PATENT SPECIFICATION

(11) **EP 1 899 328 B1**
(45) Date of publication and mention of the grant of the patent: **04.03.2009**
(21) Application number: 06754522.8
(22) Date of filing: 22.06.2006
(51) Int. Cl.: C07D 403/10

(54) **A PROCESS FOR THE PREPARATION OF LOSARTAN DERIVATIVES BY CHLORINATION AND REDUCTION OF THE RESPECTIVE 1H-IMIDAZOLE-5-CARBALDEHYDES**
VERFAHREN ZUR HERSTELLUNG VON LOSARTANDERIVATEN DURCH CHLORIERUNG UND REDUKTION DER ENTSPRECHENDEN 1H-IMIDAZOL-5-CARBALDEHYDE
PROCEDE DE FABRICATION DE DERIVES DU LOSARTAN PAR CHLORATION ET REDUCTION DES 1H-IMIDAZOLE-5-CARBALDEHYDES RESPECTIFS

(30) Priority: 04.07.2005 EP 05014451
(43) Date of publication of application: 19.03.2008
(73) Proprietor: KRKA, D.D., Novo Mesto, 8501 Novo mesto (SI)
(72) Inventor: JORDAN, Berta, Kotar, 847 01 Bratislava (SK); PUTALA, Martin, 851 01 Bratislava (SK); BARTOVIC, Alexander, 847 01 Bratislava (SK); VEVERKOVA, Eva, 851 01 Bratislava (SK); KRIZ, Miroslav, 847 01 Bratislava (SK); VEVERKA, Miroslav, 8311 Kostanjevica na Krki (SI)
(74) Representative: HOFFMANN EITLE
(86) International application number: PCT/EP2006/006040
(87) International publication number: WO 2007/003280

(56) References cited:
- EP-A- 0 535 465
- EP-A- 1 548 009
- WO-A-03/093262
- EDMUNDS ET AL: "Derivatives of 5-[[1-4(4-carboxybenzyl)imidazolyl]methyli dene]hydantoins as orally active angiotensin II receptor antagonists" JOURNAL OF MEDICINAL CHEMISTRY, vol. 38, no. 19, 1995, pages 3759-3771, XP002356656

## Description

### Field of the Invention

The present invention relates to a process for the preparation of certain sartan derivatives, particularly losartan, and novel intermediates useful in such process.

Losartan is chemically defined as 2-butyl-4-chloro-5-(hydroxymethyl)-1-{[2'-(1*H*-tetrazol-5-yl) biphenyl-4-yl]methyl}imidazole. It has the formula (I) depicted bellow with R=n-butyl. Losartan is an effective inhibitor for angiotensin-converting enzymes and is therefore widely used for the treatment of hypertension, renal failure, glaucoma and related diseases and conditions. As an angiotensin II receptor antagonist, losartan avoids the side-effects of calcium antagonists and shows high stability and obvious curative effects. 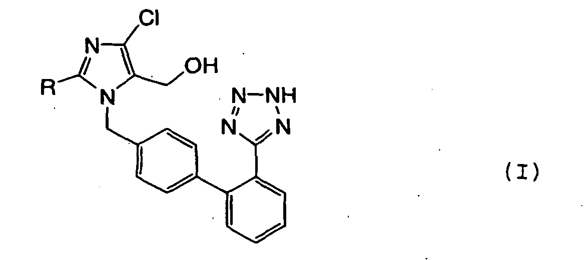 In the context of this invention, the term "losartan" also includes pharmaceutically acceptable hydrates and solvates, and the term of "a compound having formula (I)" is to be construed accordingly.

### Background of the Invention

Various approaches are known which describe the synthesis of 1,2,4,5-substituted 1*H*-imidazole-5-hydroxymethyl compounds, being useful intermediates in the synthesis of losartan.

Thus, US 4,355,004, JP 98270 and Dzurion, P. et al., W. Arch Pharmacy 1974, 307, 470 describe the synthesis of substituted 1*H*-imidazole-5-hydroxymethyl intermediates, which are involved in the synthesis of alkyl imidates or alkyl amidines. Within this reaction an extremely high-pressure (420 psi) is used in liquid ammonia and 1,3-dihydroxyacetone. Said harsh reaction conditions used in the high-pressure condensation of substituted imidate esters result in unsatisfying low and variable yields between 0-30% (Wittenberger, S.J. et al. Synthetic Commun.1993, 23, 3231).

US 5,310,928 and Larsen, D.R. et al., J. Org. Chem. 1994, 59, 6391 disclose a process in which a direct alkylation of 1*H-*imidazole derivatives is performed, however leading to practical difficulties and unselective product formation.

Further, Shi et al., Synthetic Commun. 1993, 23, 2623 describe a synthetic route for 2-butyl-4-hydroxymethyl-1*H-*imidazole starting from valeroimidate *via* valeroamidine in the presence of a co-solvent, which allows to reduce the reaction pressure. However, the synthesis of large quantities of material using such a method is cost ineffective and not reproducible.

EP-A-0 059 156 also discloses a process for preparing imidazole acetic acid intermediates. Moreover, EP 0 253 310 describes a process for the preparation of an antihypertensive compound. The synthesis of said compounds, comprising a phenylimidazole core structure, is performed by reacting a benzyl amidine with a α-haloaldehyde. However, these and the other known synthetic procedures leading to substituted 1*H*-imidazole-5-hydroxymethyl compounds, generally have relatively low yields and show a low regioselectivity. Therefore, at least some of them result in the loss of up to one-half of the material under certain circumstances. The product mixtures (regioisomers) obtained by said procedures require further separation steps, e.g. by chromatography or crystallization, causing additional costs. Therefore, the synthesis of imidazole compounds as a starting material is inconvenient and cost-intensive.

US patent 4,194,049 discloses a process for the preparation of 5-formylimidazole derivatives and intermediates thereof *via a* condensation reaction between a halomalonaldehyde and N-arylamidine, leading to the formation of one single 5-regioisomer. Its use in the synthesis of Eprosartan and its derivatives is described by the SmithKline Beecham group (Shilcrat S.C., et al., J. Org. Chem. 1997, 62, 8449) in 33 to 82% yields. The reaction of N-monosubstituted amidines with 2-halo-3-alkoxypropenals according to said publication is described as being regioselective. However, the regioselectivity of this reaction strongly depends on the substituents which can lead to the formation of undesired regioisomers of up to 15%. The reaction is performed in a mixture of water and an organic solvent, and a specific ratio of these solvents must be strictly maintained. Water is needed to solubilize the acid scavenger, but on the other hand can lead to decomposition of the reactants. Moreover, the imidazole moieties had to be protected in a specific way to avoid side-reactions.

This invention has as an object to overcome the above disadvantages and to provide the desired intermediate products, and particularly the end product losartan, in a cost-effective way and in high yields.

Through exhaustive and laborious research the inventors surprisingly recognized that suitably protected losartan intermediates can be prepared and converted into losartan with excellent yields.

### Summary of the Invention

Thus, in one aspect, the present invention provides a process for the preparation of sartan derivatives of formula (I), and particularly losartan and/or its potassium salts: 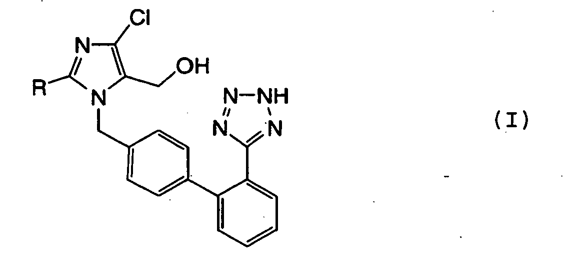 wherein R is C2-C7 straight or branched alkyl, preferably straight C3-C5 alkyl and most preferably n-butyl, or is C3-C9 cycloalkyl, such as cyclopropyl, cyclobutyl, cyclopentyl, cyclohexyl, cycloheptyl, cyclooctyl and cyclononyl, most preferably cyclopentyl, Most preferably, R is n-butyl.

The individual steps to organize the synthesis of formula (II), comprise, inter alia, the steps of:
chlorinating and reducing, in any order, a compound of formula (III): 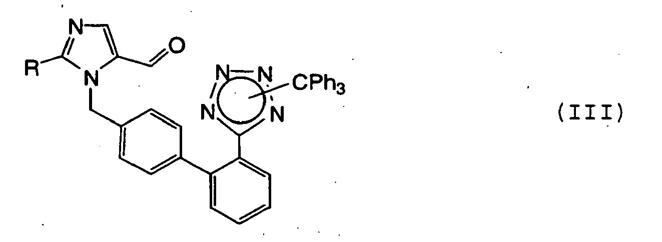
wherein R is defined as above
to form a compound of formula (VI), 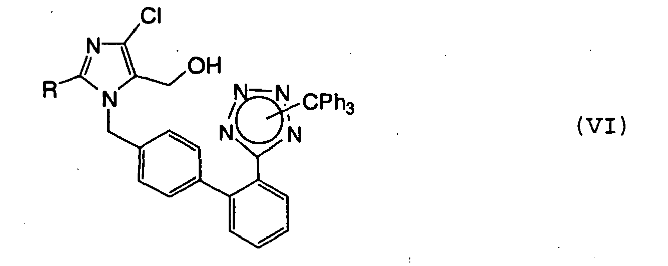 wherein R is defined as above
and then deprotecting said compound of formula (VI) to obtain the sartan derivative of formula (I), preferably losartan, and optionally converting said sartan derivative into one of its pharmaceutically acceptable salts, most preferably losartan potassium.

The order of the reducing and chlorination steps is not limited, but preferably the reduction step is carried out prior the chlorination step. Preferably deprotection is the last step.

Preferably, the chlorination reagent used in the chlorination step is an N-chloroimide such as N-chlorosuccinimide, or is 1,3-dichloro-5,5-dimethylhydantoin, 1,3-dichlorohydantoin, NaOCl, BuOCl or t-BuOCl. Even more preferably, the chlorination is carried out in THF or dimethylacetamide.

In a further aspect, the invention provides a process as described above which, as a preceding step, further comprises reacting an N-substituted amidine of formula (IV) 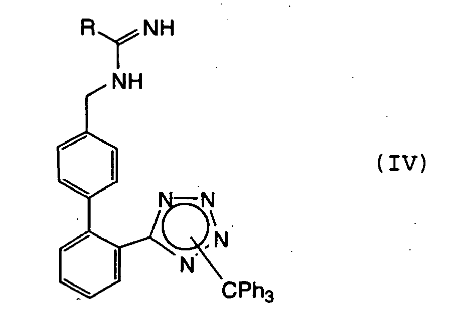 wherein R is as defined for formula (I) with 2-chloromalonaldehyde, 2-bromomalonaldehyde or, in a preferred embodiment, with 2-bromo-3-(1-methylethoxy)-2-propenal in the presence of a base in an organic solvent to form a compound of formula (III). Preferably, potassium carbonate or triethylamine are used as a base in this process step. The solvent can be dimethylformamide, dimethylsulfoxide or an alcohol selected from methanol, ethanol, n-propanol, isopropanol, n-butanol, isobutanol or tert-butanol. Dimethylformamide is preferred.

The invention also provides a novel intermediate of formula (IV) as described above and its use in the preparation of sartan derivatives, particularly losartan.

### Brief Description of the Drawings

Fig. 1 shows an FT-IR spectrum of a material as prepared according to Example 9A.
Fig. 2 shows an FT-IR spectrum of a material as prepared according to Example 9B.
Fig. 3 shows an FT-IR spectrum of a material as prepared according to Example 9C.
Fig. 4 shows an FT-IR spectrum of a material as prepared according to Example 10A.
Fig. 5 shows an FT-IR spectrum of a material as prepared according to Example 10B.
Fig. 6 shows an FT-IR spectrum of a material as prepared according to Example 10C.
Fig. 7 shows an FT-IR spectrum of a material as prepared according to Example 10D.

### Detailed Description

The various reaction steps and intermediates that can be used in the preparation of sartan derivatives, particularly losartan, are now described in greater detail with reference to preferred embodiments.

In one preferred embodiment, the present invention provides a process for the preparation of certain sartan derivatives of the formula (III), in particular the compound 2-n-butyl-1-{[2'-(2-triphenylmethyl-2*H*-tetrazol-5-yl)-1,1'-biphenyl-4-yl]methyl}-1*H*-imidazole-5-carbaldehyde: 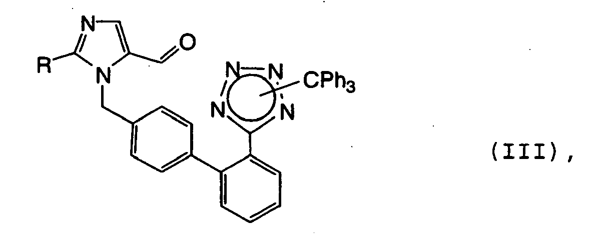 wherein R is as defined above, and preferably n-butyl by reacting an N-substituted amidine of formula (IV) 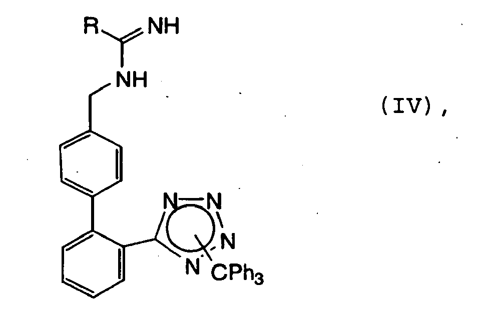 wherein R is C2-C7 straight or branched alkyl, preferably C3-C5 straight alkyl, and most preferably n-butyl, or is C3-C9 cycloalkyl, such as cyclopropyl, cyclobutyl, cyclopentyl, cyclohexyl, cycloheptyl, cyclooctyl and cyclononyl, preferably cyclopentyl,
with 2-bromo-3-(1-methylethoxy)-2-propenal, 2-chloromalonaldehyde or 2-bromomalonaldehyde in the presence of a base in an organic solvent.

Preferably, the reaction is carried out by reacting a substituted amidine of formula (IV) with 2-bromo-3-(1-methylethoxy)-2-propenal in the presence of a base, such as an inorganic base, for example sodium or potassium carbonate and an alkali metal alkoxide in an organic solvent such as dimethylformamide, dimethylacetamide, dimethylsulfoxide, or C1-C4 alcohols such as methanol, ethanol, n- and iso-propanol or a butanol. The reaction is normally carried out in a temperature range of 15°C to 95°C. Alternatively, the reaction can be carried out in the presence of an organic base and in an organic solvent. Suitable organic bases are triethylamine, diisopropylamine and buffers such as triethyl ammonium acetate. Triethylamine is particularly preferred; the preferred solvent is dimethylformamide (DMF).

It has been found that if a suitably substituted unsaturated α-halo-carbaldehyde is reacted with the amidine compound of formula (IV) according to the procedure described above, the desired 5-imidazole-carbaldehydes can be obtained in the correct configuration in high and reproducible yields. In other words, the reaction generally provides just the 5-isomer.

The exclusive formation of a 5-formyl-imidazole derivative with conservation of the protective group, i.e. the trityl group on the tetrazole moiety, for the next synthetic step is of high importance. The desired regioisomer is obtained in a purity of >99% (determined by HPLC or NMR) by simple crystallization. Even when water is excluded from this procedure the yield of the intermediates is high and the product can be obtained in excellent purity. The efficiency of the process as well as the purity and yield of the imidazole intermediates are of particular importance when preparing such a compounds on a large scale for therapeutic use.

If R in formula (IV) is n-butyl, then the substituted amidine of formula (IV) can be prepared by reaction of 5-(4'-aminomethyl-1,1'-biphenyl-2-yl)-2- or 3-triphenylmethyl-2*H-*tetrazole of formula (V) 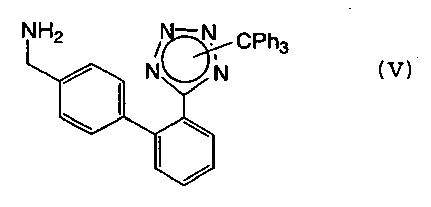 with butyl alkylimidates (butyl-C(=NH)-O-C1-C4 alkyl), for example ethyl valerimidate, prepared according to Mac Elvain et al., J. Amer. Chem. Soc. 1942, 64, 1825. The butylimidate (butyl-C(=NH)-O-C1-C4 alkyl) is either used as its hydrochloride or in the free base form. Analogous reactions can be carried out with other alkyl alkylimidates.

An N-monosubstituted amidine of formula (IV) can also be obtained according to the procedure described by Shriner, R.L, et al., Chem. Rev. 1944, 35, 351, or analogous methods.

The preferred starting material 2-bromo-3-(1-methylethoxy)-2-propenal can be prepared according to the procedure described in US 4,194,049 (Example 3) by halogenation of malonaldehyde bis-dialkyl acetal, followed by O-alkylation.

After a reaction time of 2 to 16 hours, the resulting compound according to formula (III) can be isolated from the reaction mixture in a manner known to those skilled in the art, but preferentially by precipitating it from the reaction mixture by addition of water. The molar ratio of 2-bromo-3-(1-methylethoxy)-2-propenal to the substituted amidine of formula (IV) is between 1 and 1.5.

The compound of formula (III) can then be further reacted by chlorination with a suitable chlorinating agent selected from the group of N-chloroimides, such as N-chlorosuccinimide or 1,3-dichloro-5,5-dimethylhydantoin, 1,3-dichlorohydantoin, NaOCl, BuOCl or t-BuOCl in a suitable solvent such as DMF, DMSO, dimethylacetamide, THF and a C1-C4 alcohol, preferably dimethylacetamide. This reaction yields a compound of formula (II) 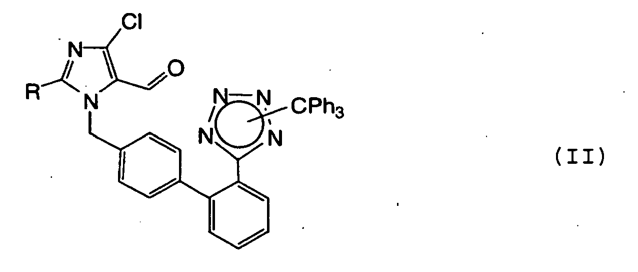 wherein R is as defined above, which can then be further reacted with a suitable reducing agent such as sodium borohydride, lithium aluminium hydride or NaH/DMF, to form the corresponding alcohol. The order of the chlorination and the reduction step can also be reversed, and it is even preferred to conduct the reduction before the chlorination step. As a last step, this intermediate can be deprotected under acidic conditions, e.g. 10% aq. hydrochloric acid in THF, to form a sartan derivative of formula (I), preferably losartan. A reaction of this type is described, *inter alia,* in J. Med. Chem. 1991, 34, 2525-2547.

Optionally, the sartan derivative of formula (I) can then be converted into one of its pharmaceutically acceptable salts, such as a pharmaceutically acceptable alkaline or earth alkaline or amine salt (e.g. the sodium salt), or an acid addition salt (such as the hydrochloride). Losartan and its potassium salt are particularly preferred, but such salts and methods of their preparation are generally known to one skilled in the art.

The invention is illustrated by the following examples. The examples do not intend to limit the scope of this invention as defined in the claims below.

### Examples

### Example 1

### A) Preparation of 5-(4'-phthalimidomethyl-1,1'-biphenylyl)-2-triphenylmethyl-2H-tetrazole

A mixture of 5.50 g (0.01 mol) of 5-(4'-bromomethyl-1,1'-biphenyl-2-yl)-2-triphenylmethyl-2*H*-tetrazole and 2.20 g of potassium phthalimide in 20 mL anhydrous dimethylformamide was stirred at room temperature for 48 hours. The mixture was added to 300 mL of water with vigorous stirring. The solid was filtered off, washed with 20 mL water and dried at a temperature of 45°C. 6.00 g of the product could be obtained as a white solid with a m.p. of 167-172°C.
After re-crystallization of the crude solid from a mixture of toluene and cyclohexane 5.40 g of the product could be obtained as white crystals with a m.p. of 171-173°C.

### B) Preparation of 5-(4'-aminomethyl-1,1'-biphenyl-2-yl)-2-triphenylmethyl-2H-tetrazole

8.90 g of 5-(4'-phthalimidomethyl-1,1'-biphenyl-2-yl)-2-triphenylmethyl-2*H*-tetrazole were dissolved in 50 mL of dioxane. 2.90 mL of 100% hydrazine hydrate was added in one portion. The resulting reaction mixture was refluxed for 4 hours with vigorous stirring, cooled down to 40-50°C and then reacted with 8.50 mL of 20% aq. sodium hydroxide. The reaction mixture was stirred at room temperature for 12 hours. The solvent was removed by vacuum distillation and the resulting raw product dissolved in 100 mL of hot water (t = ∼60°C). Finally the mixture was extracted twice with 30 mL of ethyl acetate. The combined organic phases were dried over anhydrous sodium sulfate and ethyl acetate was removed by vacuum distillation. Recrystallization from toluene-cyclohexane yielded in 5.50 g product in form of a white powder with a m.p. of 143-144°C.

### C) Preparation of methyl valerimidate hydrochloride

To a solution of 10.1 g of valeronitrile in 4.50 mL of anhydrous methanol gaseous hydrochloric acid was introduced under external cooling (ice/sodium chloride mixture). The hydrogen chloride was bubbled at a rate such that the temperature of the reaction mixture remained below 15°C. After approximately 45 minutes 10.0 g of hydrogen chloride were absorbed. After placing a lid on the reaction flask it was put into the refrigerator overnight. Tert-butyl methyl ether was added (60 mL) to the reaction mixture with stirring. The resulting voluminous suspension was then filtered off with a flask-to-flask assemble. After drying under vacuum at ambient temperature 18.1 g of the product with a m.p. of 96-98°C could be obtained.

### D) Preparation of N-{[2'-(2-triphenylmethyl-2H-tetrazol-5-yl)-1,1'-biphenyl-4-yl]methyl}pentaneamidine

To a stirred solution of 3.18 g (0.021 mol) of methyl valerimidate hydrochloride in 25 mL of anhydrous dimethylformamide, a solution containing 4.33 g of triethylamine in 7.00 mL dimethylformamide was added. The temperature was maintained at 0°C. The resulting suspension was stirred for 1 hour at ambient temperature and then filtered with 7.00 mL of dimethylformamide. To the combined filtrates a solution containing 7.80 g of 5-(4'-aminomethyl-1,1'-biphenyl-2-yl)-2-triphenylmethyl-2*H-*tetrazole in 40 ml dimethylformamide was added under nitrogen. The reaction was performed in a nitrogen atmosphere with stirring for 1 hour at ambient temperature, and then heated to 60°C for 16 hours. The reaction mixture was added to a sodium chloride solution (500 mL) with vigorous stirring. After filtration and drying, 7.80 g of a yellowish mass, was obtained. Then a mixture of 60 mL of tert-butyl methyl ether and 6.00 mL of toluene was added, stirred for 0.5 hour and then filtered. The product was dried at ambient temperature to provide 6.20 g of a yellow crude product.
¹H-NMR (DMSO-d6, 300 MHz): 7.64 (1H, m, 7.60-7.65), 7.55 (1H, m, 7.5-7.60), 7.46 (1H, m, 7.40-7.50), 7.35 (9H, m, 7.30-7.40), 7.23 (2H, m, 7.21-7.24), 7.08 (2H, m, 7.06-7.1), 6.89 (6H, m, 6.85-6.92), 4.34 (2H, s, CH2), 2.33 (2H, t), 1.58 (2H, m), 1.29 (2H, m), 0.89 (3H, t)

### E) Preparation of 2-n-butyl-1-[(2'-(2-triphenylmethyl-2H-tetrazol-5-yl)-1,1'-biphenyl-4-yl)methyl)-1H-imidazole-5-carbaldehyde

To a mixture of 5.20 g of N-{[2'-(2-triphenylmethyl-2*H-*tetrazol-5-yl)-1,1'-biphenyl-4-yl]methyl}pentanamidine and 1.50 g potassium carbonate in 60 mL dimethylformamide a solution of 2-bromo-3-(1-methylethoxy)-2-propenal (2.0 g) in 20 ml of dimethylformamide was added at 0°C within 0.5 hours. The reaction mixture was stirred under nitrogen at 0°C for 2 hours, then heated up to 60°C and stirred for another 8 hours. The reaction mixture was poured into a sodium chloride solution (400 mL). The precipitated yellow product was separated by suction and washed with 10 mL of water. After filtration and drying, 5.00 g of a slightly yellowish mass was obtained. The resulting crude product was then suspended twice in 60 mL tert-butyl methyl ether, filtered off and dried to obtain a pale yellowish powder in an amount 4.70 g (82%). A sample of the product was crystallized from ethanol. A hot cloudy solution was filtered, cooled down to obtain a pale yellowish crystalline solid with a m.p. of 143-145°C.
¹H-NMR (CDCl₃, 300 MHz): 9.65 (1H, s, CHO), 7.87 (1H, m 7.80-7.94), 7.78 (1H, s, H on imidazole ring), 7.46 (2H, m, 7.40-7.52), 7.35 (4H, m, 7.30-7.40), 7.25 (6H, m, 7.20-7-30), 7.08 (2H ,m, 7.06-7.09), 6.9(6H, m, 6.86-6-94), 6.82 (2H,m, 6.78-6.86), 5.47 (2H, s, CH2), 2.55 (2H, t), 1.64 (2H, m), 1.29 (2H, m), 0.86 (3H, t)

### Example 2

### Preparation of 2-n-butyl-1-[(2'-(2-triphenylmethyl-2H-tetrazol-5-yl)-1,1'-biphenyl-4-yl) methyl]-1H-imidazole-5-carbaldehyde

0.1 g of N-{[2'-(2-triphenylmethyl-2*H*-tetrazol-5-yl)-1,1'-biphenyl-4-yl]methyl}pentanamidine was added to 0.04 g of potassium carbonate. To this mixture 2 mL of dimethylsulfoxide and 0.04 g of 2-bromo-3-(1-methylethoxy)-2-propenal were added. The reaction mixture was stirred under nitrogen at a temperature of 40°C overnight.

To the reaction mixture was added 2.00 ml of water and the resulting solid was filtered off. The precipitated mass was suspended in 4.00 ml of diethyl ether, filtered and then washed with 2.00 ml of diethyl ether. 0.08 g (73%) of a slightly brown powder was obtained with a m.p. of 141-143°C, HPLC assay 99%.

### Example 3

### Preparation of 2-n-butyl-1-[(2'-(2-triphenylmethyl-2H tetrazol-5-yl)-1,1'-biphenyl-4-yl)methyl]-1H-imidazole-5-carbaldehyde

Analogous to example 2, but instead of potassium carbonate 0.30 g of triethylamine and 2.00 ml of 2-propanol were used.

### Example 4

### Preparation of 2-n-butyl-1-[(2'-(2-triphenylmethyl-2H-tetrazol-5-yl)-1,1'-biphenyl-4-yl)methyl]-1H-imidazole-5-carbaldehyde

Analogous to example 2, but instead of potassium carbonate 0.30 g triethylamine and 0.17 g of acetic acid were used.

### Example 5

### Preparation of 2-n-butyl-1-[(2'-(2-triphenylmethyl-2H-tetrazol-5-yl)-1,1'-biphenyl-4-yl)methyl]-1H-imidazole-5-carbaldehyde

Analogous to example 2, but instead of 2-bromo-3-(1-methylethoxy)-2-propenal 0.02 g of 2-chloromalonaldehyde was used.

### Example 6

### A) Preparation of 2-n-butyl-1-{2'-[2-(triphenylmethyl)-2H-tetrazol-5-yl]-1,1'-biphenyl-4-yl}-1H-imidazole-5-methanol

The suspension of 2-n-butyl-1-{2'-[2-(triphenylmethyl)-2*H-*tetrazol-5-yl]-1,1'-biphenyl-4-yl}-1*H*-imidazole-5-carboxaldehyde 1.229 g in methanol (100 mL) was cooled to 0°C and then NaBH₄ 0.076 g was added portion-wise over 30 min. The mixture was stirred 1 hour and then warmed to ambient temperature. After additional 2 hours the cloudy reaction mixture was filtered off and was concentrated to 1/5 of the original volume. The residue was quenched with a saturated solution of ammonium chloride and extracted with ethyl acetate (2x50mL). The organic layer was washed with water, brine and dried over Na₂SO₄. The solvent was removed and the product was obtained in 85.2% yield (1.05 g). The structure confirmed by ¹H NMR.

### B) Preparation of 2-n-butyl-4-chloro-1-{2'-[2-(triphenylmethyl)-2H-tetrazol-5-yl]-1,1'-biphenyl-4-yl}-1H-imidazole-5-methanol

2-n-butyl-1-{2'-[2-(triphenylmethyl)-2*H-*tetrazol-5-yl]-1,1'-biphenyl-4-yl}-1*H*-imidazole-5-methanol (100 mg) was dissolved in dry THF (3 mL). To the stirred solution was added N-chlorosuccinimide (31.7 mg) over 2 hours (each 30 min about ¼ of amount of N-chlorosuccinimide) at room temperature. The mixture was stirred for additional two hours and stopped when the formation of by-products was observed by TLC (ethyl acetate: hexane, 1:1). The solvent was removed under reduced pressure and residue was precipitated with brine (2 mL). The solid was filtered off and dried to obtain 78 mg of crude product.

### Example 7

### A) Preparation of 2-n-butyl-4-chloro-1-{[2'-(2-triphenylmethyl-2H-tetrazol-5-yl)-1,1'-biphenyl-4-yl]methyl}-1H-imidazole-5-carbaldehyde

300 mg of 2-n-butyl-1-{[2'-(2-triphenylmethyl-2*H*-tetrazol-5-yl)-1,1'-biphenyl-4-yl]methyl}-1*H*-imidazole-5-carbaldehyde was dissolved in 20 mL of dimethylacetamide and the reaction mixture was heated to 40°C. At this temperature 95.6 mg of N-chlorosuccinimide were added to the reaction mixture. The mixture was stirred at 40°C for 2.5 hour. After 2.5 hours 31.8 mg of N-chlorosuccinimide was added. The reaction mixture was stirred at 40°C for further 2.5 hours. After cooling to ambient temperature the reaction was poured dropwise to a solution containing 20 mL of water, 10 mL of saturated NaCl solution and 100 mg of NaHCO₃. The mixture was stirred at ambient temperature 0.5 hours and the precipitated crude product was filtered off, washed with 50 mL of water and dried to obtain 278 mg (87.8%) of a white solid.

### B) Preparation of 2-n-butyl-4-chloro-1-{2'-[2-(triphenylmethyl)-2H-tetrazol-5-yl]-1,1'-biphenyl-4-yl}-1H-imidazole-5-methanol

A suspension of 278 mg of 2-n-butyl-4-chloro-1-{2'-[2-(triphenylmethyl)-2*H*-tetrazol-5-yl]-1,1'-biphenyl-4-yl}-1*H-*imidazole-5-carboxaldehyde in methanol (15 mL) was cooled to 0°C and reacted with 0.016 g of NaBH₄. The mixture was stirred 1 hour at 0°C and then warmed to ambient temperature. After additional 2 hours the mixture was filtered and the solvent was evaporated. The residue was quenched with a saturated solution of ammonium chloride and extracted with ethyl acetate (2x15mL). The organic layer was washed with water, brine and dried over Na₂SO₄. The solvent was removed and the product was dried under reduced pressure to obtain title product.

### Example 8

### Preparation of 2-n-butyl-4-chloro-1-{[2'-(2-triphenylmethyl-2H-tetrazol-5-yl)-1,1'-biphenyl-4-yl]methyl}-1H-imidazole-5-carbaldehyde

30 mg of 2-n-butyl-1-{[2'-(2-triphenylmethyl-2*H-*tetrazol-5-yl)-1,1'-biphenyl-4-yl]methyl}-1*H*-imidazole-5-carbaldehyde was dissolved in 2 mL of dichloromethane and then reacted with 30.5 mg of 14% sol. of sodium hypochlorite in water. The mixture was stirred at 40°C for 24 hours.

After consumption of the starting material the reaction mixture was washed with water (2x3mL) and then dried over Na₂SO₄. The solvent was removed and the product was dried under reduced pressure to obtain the title product in 88.6% yield (28 mg). The structure was confirmed by ¹H NMR.

### Example 9

### A) Crystallisation of 2-butyl-4-chloro-1-[[2'-(2-triphenylmethyl-2H-tetrazol-5-yl)-1,1'-biphenyl-4-yl]methyl}-1H-imidazole-5-methanol (solvated trityl losartan)

27.7 g of wet trityl losartan were suspended in ethyl acetate (95 mL) and heated to reflux. The hot solution was filtered under vacuum and after cooling the product rapidly crystallised. The suspension was stirred for an additional half an hour at 15°C. The product was filtered off, washed with 8 mL of ethyl acetate and dried in a vacuum dryer at 40°C to obtain the solvated form of trityl losartan in 19.1 g yield. M.p.:88-90°C

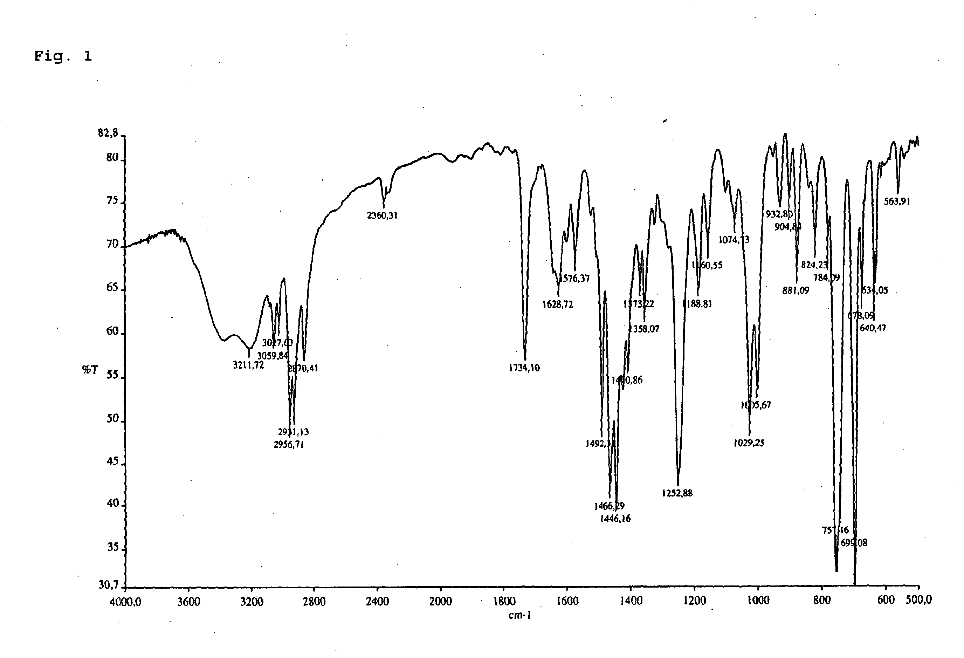

The FT-IR spectrum of the obtained material is shown in Fig. 1.

### B) Crystallisation of 2-butyl-4-chloro-1-[[2'-(2-triphenylmethyl-2H-tetrazol-5-yl)-1,1'-biphenyl-4-yl]methyl}-1H-imidazole-5-methanol (anhydrous trityl losartan)

35.2 g of wet trityl losartan were suspended in ethyl acetate (95 mL), heated to reflux and stirred for 10 min. Then the cloudy solution was gradually cooled to 15°C. The suspension was stirred for an additional half an hour at 15°C. The product was filtered, washed with 8 mL of ethyl acetate and dried in a vacuum dryer at 40°C to obtain the anhydrous form of trityl losartan in 22.2 g yield. M.p. 171-174°C

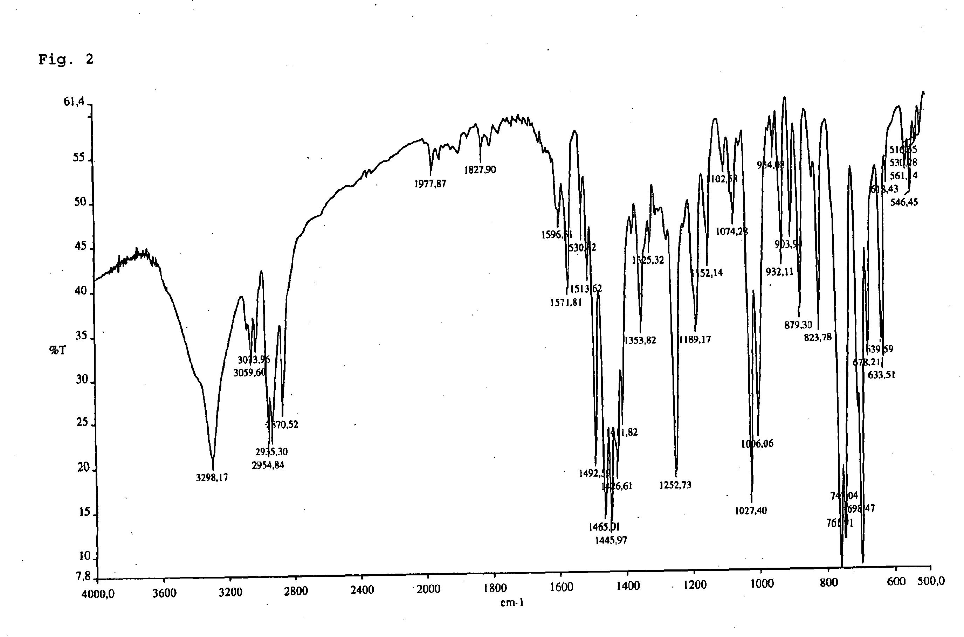

The FT-IR spectrum of the obtained material is shown in Fig. 2.

### C) Drying of solvated 2-butyl-4-chloro-1-[[2'-(2-triphenylmethyl-2H-tetrazol-5-yl)-1,1'-biphenyl-4-yl]methyl}-1H-imidazole-5-methanol to obtain the anhydrous form of trityl losartan

Drying the solvated trityl losartan in vacuum under 50 mbar and gradually heating to 50°, 70°C and to 100°C yielded the anhydrous form of trityl losartan.M.p. 167-171°C

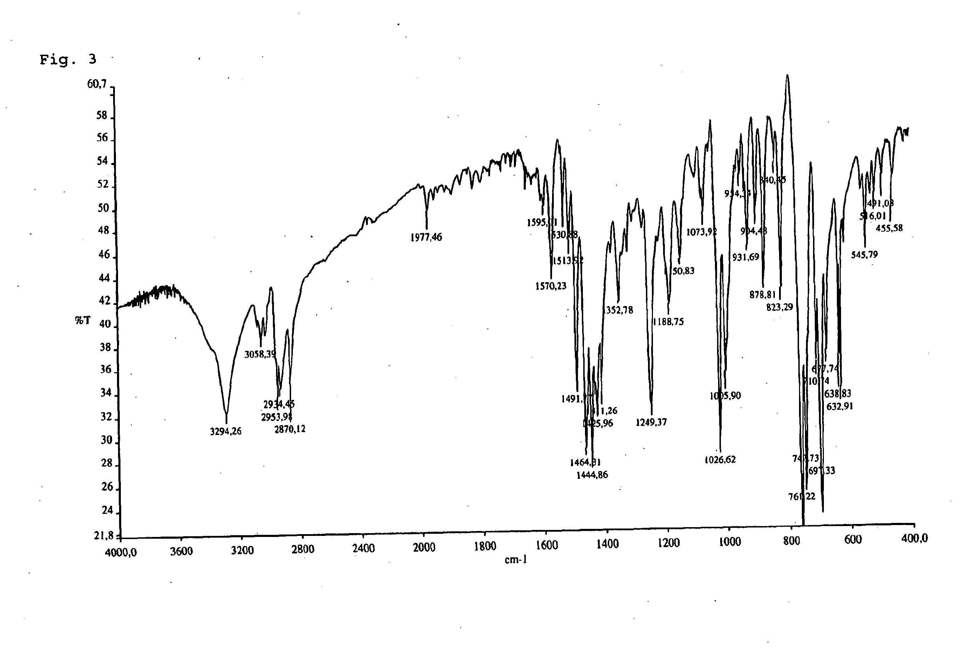

The FT-IR spectrum of the obtained material is shown in Fig. 3.

Alternatively other acetates as iso-propyl acetate, butyl acetate, or ketones such as acetone, ethyl methyl ketone, iso-butyl methyl ketone, or mixtures of a chlorinated alkanes and water, such as butyl chloride/water, are used for the crystallisation of trityl losartan.

### Example 10

### A) Preparation of 2-butyl-4-chloro-1-[2'-(1H-tetrazol-5-yl)(1,1'-biphenyl)-4-yl]methyl-1H-imidazole-5-methanol (solvated losartan)

32.0 g of 2-butyl-4-chloro-1-{[2'-(2-triphenylmethyl-2*H-*tetrazol-5-yl)-1,1'-biphenyl-4-yl] methyl}-1*H*-imidazole-5-methanol (trityl losartan) were suspended in acetonitrile (114 mL) and gradually reacted with 12% hydrochloric acid (48.4 mL). The mixture was stirred for about 5 hours at 18-20°C. The pH of the mixture was adjusted to 12.5 ± 0.2 with 30% sodium hydroxide solution (24 mL). The acetonitrile was removed and replaced by addition of water. After cooling down to room temperature the insoluble triphenylmethanol was removed by centrifugation and washed with water. Ethyl acetate (51 mL) was added to the aqueous solution and the pH of the solution was adjusted to 3.8 ± 0.2 with conc. hydrochloric acid. The mixture was cooled below 10°C and left at this temperature for not less than 1 hour.

The crystallized product was collected after centrifugation and washed with a water-methanol mixture followed by a washing step with ethyl acetate. The product was dried in a dryer below 50°C until the water content (KF) was not more than 0.2%. The solvated product was obtained in 90.5% yield (18.4 g). M.p. 115-123°C

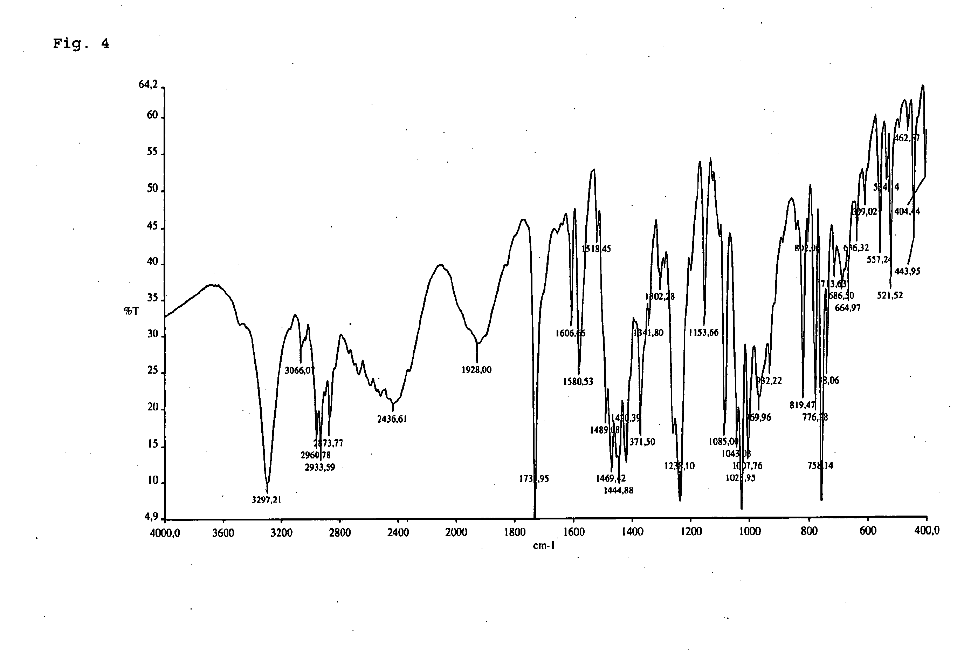

The FT-IR spectrum of the obtained material is shown in Fig. 4.

### B) Crystallisation of 2-butyl-4-chloro-1-[2'-(1H-tetrazol-5-yl)(1,1'-biphenyl)-4-yl]methyl-1H-imidazole-5-methanol

20.34 g of losartan were suspended in water (216 mL), the pH of the mixture was adjusted to 11.5 with 30% aqueous sodium hydroxide solution and filtered to obtain a clear solution. Ethyl acetate (51 mL) was added to the aqueous solution and the pH of the solution was adjusted to 3.8 ± 0.2 with conc. hydrochloric acid. The mixture was cooled below 10°C and aged at this temperature for not less than 1 hour. The crystallized product was collected after centrifugation and washed with a water-methanol mixture followed by washing step with ethyl acetate. The product was dried in dryer below 50°C until the water content (KF) was not more than 0.2 %. The solvated ethyl acetate product was obtained in 19.2 g yield. M.p. 115-123°C.

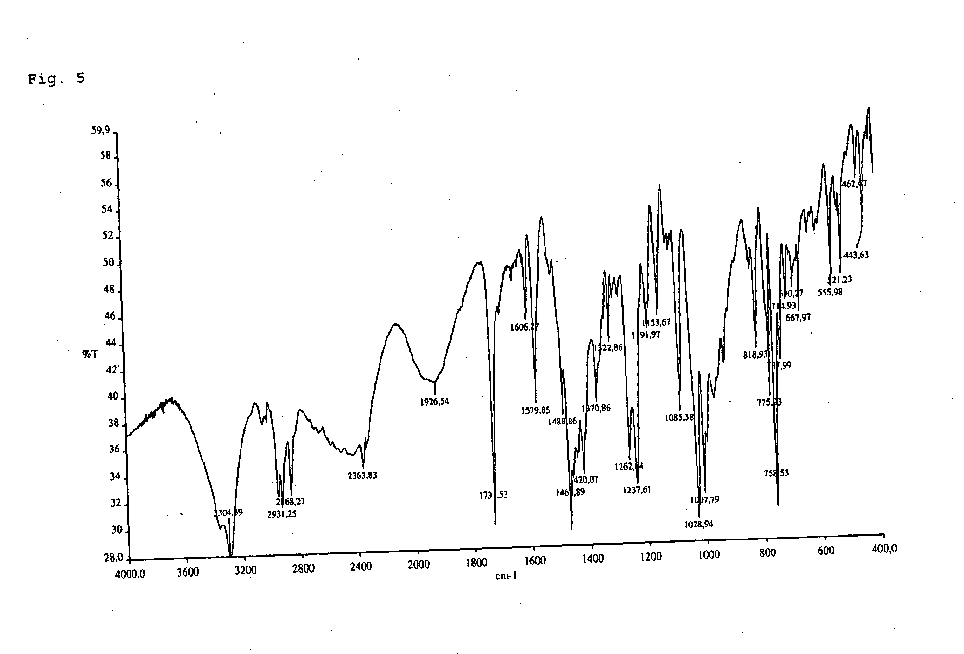

The FT-IR spectrum of the obtained material is shown in Fig. 5.

### C) Crystallisation of 2-butyl-4-chloro-1-[2'-(1H-tetrazol-5-yl) (1,1'-biphenyl)-4-yl] methyl-1H-imidazole-5-methanol (anhydrous form I of losartan)

1.0 g of losartan was suspended in acetonitrile (25 mL), heated to reflux (80°C) and filtered hot to obtain a clear solution. Then the solution was gradually cooled to room temperature. The suspension was stirred for an additional half an hour at 22°C. The product was filtered and dried in a vacuum dryer at 50°C to obtain the anhydrous form I of losartan in 0.84 g yield. M.p. 168-170°C

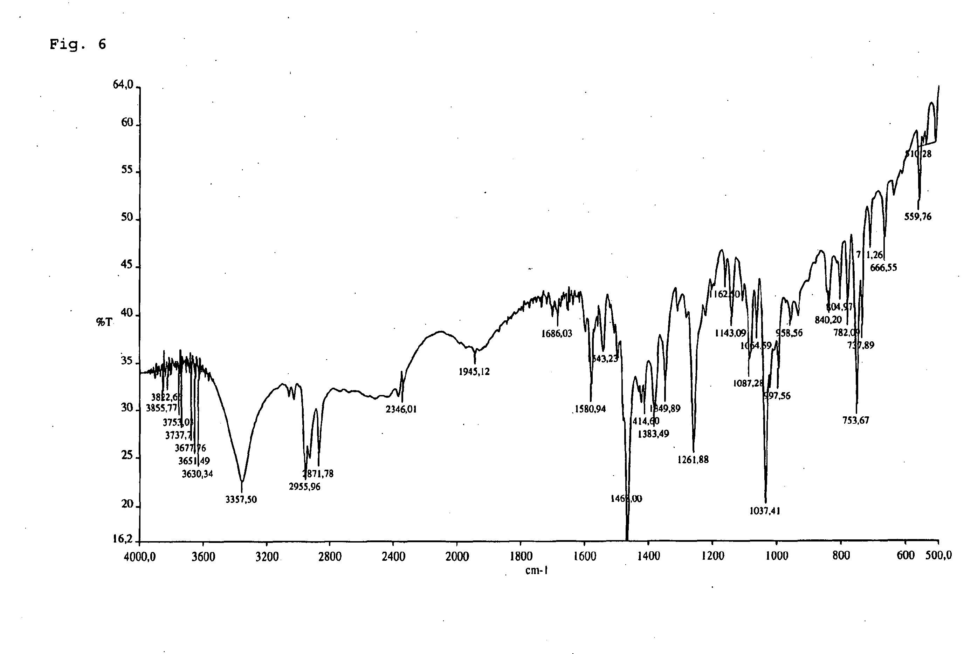

The FT-IR spectrum of the obtained material is shown in Fig. 6.

### D) Crystallisation of 2-butyl-4-chloro-1-[2'-(1H-tetrazol-5-yl)(1,1'-biphenyl)-4-yl]methyl-1H-imidazole-5-methanol (anhydrous form II of losartan)

5.0 g losartan were suspended in iso-propanol (25 mL) and heated to reflux. The suspension was stirred for one hour at that temperature (80°C) and was then gradually cooled to room temperature. The product was filtered off and dried in vacuum dryer at 50°C to obtain the anhydrous form II of losartan. In 3.50 g yield. M.p. 185-190°C

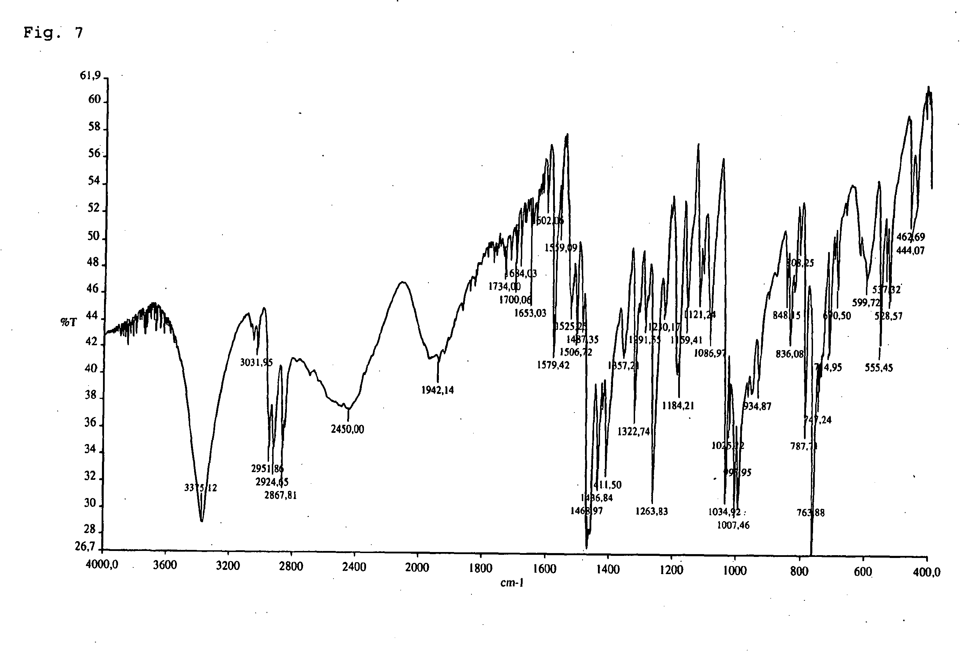

The FT-IR spectrum of the obtained material is shown in Fig. 7.

### E) Drying of solvated 2-butyl-4-chloro-1-[2'-(1H-tetrazol-5-yl)(1,1'-biphenyl)-4-yl]methyl-1H-imidazole-5-methanol to obtain the anhydrous form of losartan

Drying of solvated losartan in vacuum under 50 mbar and gradually heating to 50°, 70°C and 100°C led to the anhydrous form of losartan. M.p. 130°C, after recrystallisation m.p. 187°C, FT-IR, form II.

Alternatively other acetates such as iso-propyl acetate and butyl acetate, and alcohols such as ethanol, propanol, butanol can be used for the crystallisation (recrystallisation, maceration or precipitation) of losartan.

### Example 11

### Preparation of losartan potassium salt

2-butyl-4-chloro-1- [2'- (1*H*-tetrazol-5-yl) (1,1'-biphenyl)-4-yl]methyl-1*H*-imidazole-5-methanol (17.4 g) was dissolved in 100 mL of i-propanol and treated with 50% aq. potassium hydroxide (6.60 mL) until the constant pH was 10 ± 0.5 at 40°C. The cloudy solution was filtered and additional 100 mL of i-propanol were added to the filtrate.

The water present in the solution was removed by azeotrope distillation of i-propanol (approximately of 100 mL). If the requirement for water content (KF not more than 1.2%) is not reached, distillation is continued with addition of i-propanol. The product was crystallized by addition of heptan (40 mL) at 75-80°C.

The mixture was concentrated (aproximately 50 mL are removed) and if the requirement for water content is not reached (KF not more than 0.6%) distillation was continued with addition of heptane. The suspension was gradually cooled within 1 hour, stirred at room temperature for additional 2 hours and then the solid was collected by centrifugation. The product was dried in an dryer at 60 ± 5°C to give 17.0 g (90.0 %).

### Example 12

The following illustrates preferred chlorination conditions.

### General preparation

To a solution of 2-butyl-3-[2'-(2-trityl-2*H*-tetrazol-5-yl)-biphenyl-4-ylmethyl]-3*H*-imidazole-4-carbaldehyde (100 mg, 0.16 mmol) in 1 mL DMAA, was added dropwise at r.t. within 15 minutes a solution of reagent A in 1 mL dioxane. The reaction mixture was stirred at r.t. The amount of chlorinated product was determined by HPLC.

| **Reaction** | **Reagent A** | **Eq.** | **Time/hrs** | **Yield %** |
|---|---|---|---|---|
| 1 | Trichloromelamine | 1.5 | 24 | 46,6 |
| 2 | N-chlorophthalimide | 1.5 | 24 | 49,1 |
| 3 | Trichloroisocyanuric acid | 1.5 | 4 | 48,0 |
| 4 | Trichloroisocyanuric acid, 1-H-benzotriazole (0.65 eq.) | 1.5 | 4 | 46,2 |
| 5 | N-chlorophthalimide, 18-crown-6 (28 mol%) | 1.5 | 24 | 52,3 |
| 6 | Trichloromelamine | 2.0 | 24 | 45,8 |
| 7 | Trichloromelamine | 3.0 | 24 | 52,0 |
| 8 | N-chlorophthalimide | 2.0 | 24 | 46,5 |
| 9 | Trichloroisocyanuric acid, 0.65 eq. DBU | 2.0 | 24 | 46,5 |
| 10 | N-chlorophthalimide | 2.0 | 24 | 45,0 |
| 11 | Trichloroisocyanuric acid, 12-crown-6 (12% mol eq.) | 1.5 | 4 | 51,8 |

### Specific example

To a solution of 5.05 g 2-butyl-3-[2'-(2-trityl-2*H*-tetrazol-5-yl)-biphenyl-4-ylmethyl] -3*H*-imidazole-4-carbaldehyde in 25 mL DMAA cooled to 0°C, was added dropwise at r.t. within 15 minutes a solution of 0.948 g trichloroisocyanuric acid in 25 mL dioxane, followed by stirring at r.t. for 2 hours.

To the reaction mixture, which was cooled to 0°C, was added portionwise during 15 minutes a solution of 0.315 g NaBH₄ in 2 mL water, followed by stirring at r.t. for 2 hours. Subsequently, the reaction mixture was poured into 100 mL of NH₄Cl and extracted 3 times with 50 mL ethyl acetate. The combined organic layers were washed with 50 mL brine, dried over Na₂SO₄, followed by evaporation of the solvent. The crude product in form of an oil was obtained in 6.53 g. The oil was dissolved in 15 mL ethyl acetate, heated to reflux, cooled to r.t., and stirred for 1 hour. The precipitate was filtered off. In a first crystallization from ethyl acetate 1.54 g of the desired product was obtained.

## Claims

1. A process for the preparation of a sartan derivative of formula (I): 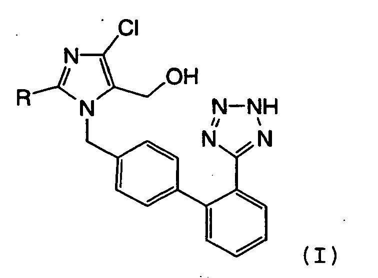 wherein R = C2-C7 straight or branched,alkyl or C3-C9 cycloalkyl,
or a pharmaceutically acceptable salt thereof, comprising the steps of:
chlorinating and reducing, in any order, a compound of formula (III): 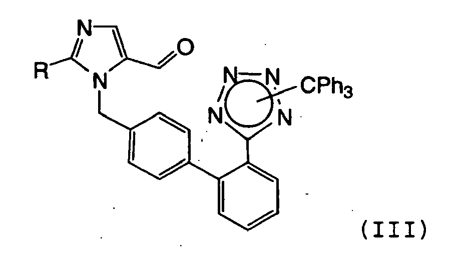
wherein R is defined as above
to form a compound of formula (VI), 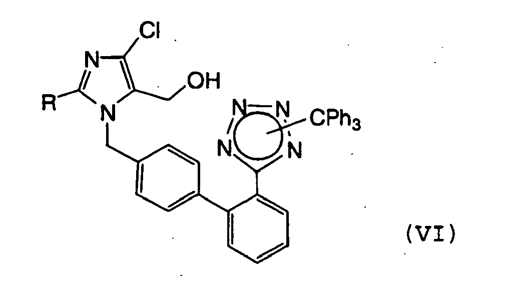 wherein R is defined as above
and then deprotecting said compound of formula (VI) to obtain the sartan derivative of formula (I) and, optionally, converting said sartan derivative into one of its pharmaceutically acceptable salts.

2. A process according to claim 1 wherein said sartan derivative is losartan.

3. A process according to claims 1 and 2, wherein said pharmaceutically acceptable salt is losartan potassium.

4. A process according to claim 1 **characterized in that** the chlorination reagent used in the chlorination step is selected from an N-chloroimide, 1,3-dichloro-5,5-dimethylhydantoin, 1,3-dichlorohydantoin, NaOCl, BuOCl, or t-BuOCl.

5. A process according to claim 4 **characterized in that** the chlorination is carried out in THF or dimethylacetamide.

6. A process according to any of claims 1-5, further comprising the step of:
reacting an N-substituted amidine of formula (IV) 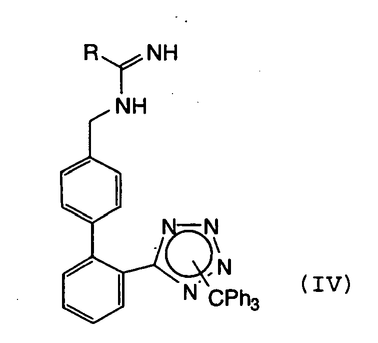
wherein R = C2-C7 straight or branched alkyl or C3-C9 cycloalkyl,
with a compound selected from 2-bromo-3-(1-methylethoxy)-2-propenal, 2-chloromalonaldehyde or 2-bromomalonaldehyde in the presence of a base in an organic solvent to form a compound of formula (III) wherein R is as defined as above.

7. A process according to claim 6 **characterized in that** the base is selected from potassium carbonate and triethylamine.

8. A process according to claim 6 or 7 **characterized in that** the solvent is dimethylformamide, dimethylsulfoxide or an alcohol selected from methanol, ethanol, n-propanol, isopropanol, n-butanol or isobutanol.

9. A process according to claim 7 **characterized in that** the solvent is dimethylformamide.

10. A process according to claim 6 further comprising the step:
converting a compound of formula (V): 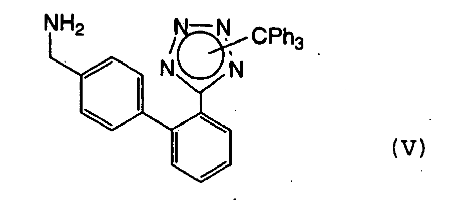
into an amidine of formula (IV).

11. A process according to any of the preceding claims, wherein the reduction step is carried out after the chlorination step.

12. A process according to any of the preceding claims, wherein the reduction step is carried out prior to the chlorination step.

13. A process for the preparation of 2-n-butyl-1-[(2'-(2- or 3-triphenylmethyl-2*H*-tetrazol-5-yl)-1,1'-biphenyl-4-yl)methyl]-1*H*-imidazole-5-carbaldehyde of the formula (IIIa) : 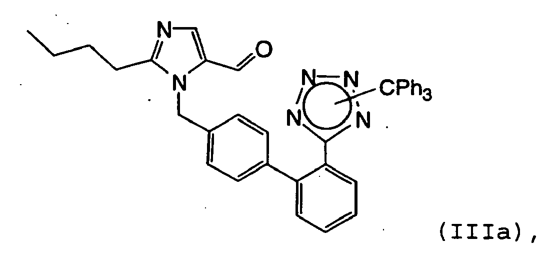 comprising reacting an N-substituted amidine of formula (IVa) 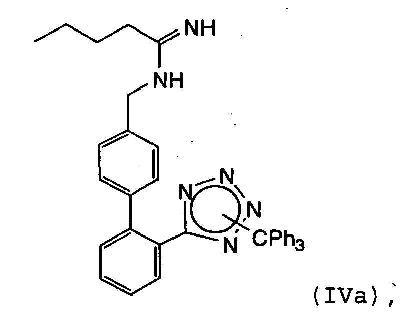 with a compound selected from 2-bromo-3-(1-methylethoxy)-2-propenal, 2-chloromalonaldehyde or 2-bromomalonaldehyde in the presence of base in an organic solvent.

14. A compound of formula (IV) or (IV)', 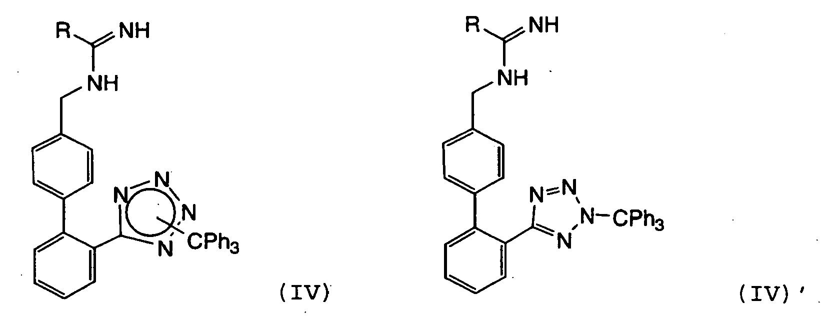 wherein R is defined as above.

15. Use of a compound according to claim 14 as an intermediate in the preparation of a sartan derivative, or a pharmaceutically acceptable salt thereof, preferably losartan or its potassium salt.

## Patentansprüche

1. Verfahren zur Herstellung eines Sartanderivats der Formel (I): 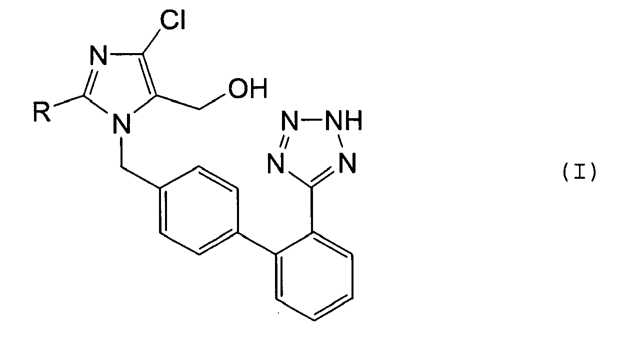 wobei R = ein C₂-C₇ geradkettiges oder verzweigtes Alkyl oder C₃-C₉-Cycloalkyl ist,
oder ein pharmazeutisch akzeptables Salz davon, umfassend die Schritte:
Chlorieren und Reduzieren einer Verbindung der Formel (III) in jeder beliebigen Reihenfolge: 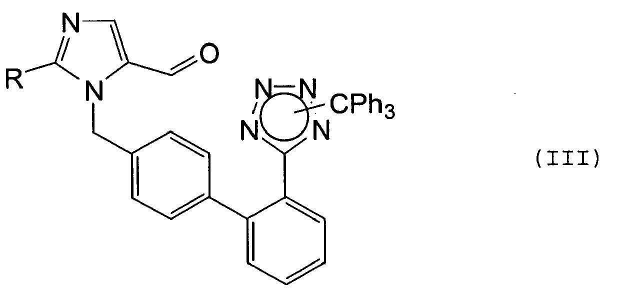
wobei R wie oben definiert ist,
um eine Verbindung der Formel (VI) zu bilden: 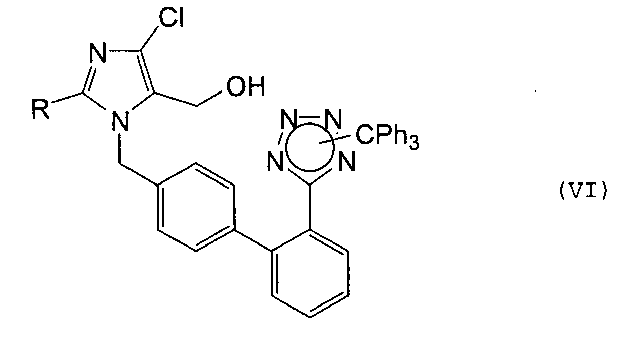 wobei R wie oben definiert ist,
und Entschützen derselben Verbindung (VI), um das Sartanderivat der Formel (I) zu erhalten und gegebenenfalls Umwandeln des Sartanderivats in eines seiner pharmazeutisch akzeptablen Salze.

2. Verfahren gemäß Anspruch 1, wobei das Sartanderivat Losartan ist.

3. Verfahren gemäß Anspruch 1 und 2, wobei das pharmazeutisch akzeptable Salz Losartankalium ist.

4. Verfahren gemäß Anspruch 1, **dadurch gekennzeichnet, daß** das Chlorierungsmittel, welches in dem Chlorierungsschritt verwendet wird, ausgewählt ist aus N-Chlorimid, 1,3-Dichlor-5,5-dimethylhydantoin, 1,3-Dichlorhydantoin, NaOCl, BuOCl oder t-BuOCl.

5. Verfahren gemäß Anspruch 4, **dadurch gekennzeichnet, daß** der Chlorierungsschritt in THF oder Dimethylacetamid durchgeführt wird.

6. Verfahren gemäß einem der Ansprüche 1 bis 5, ferner umfassend den Schritt:
Umsetzen eines N-substituierten Amidins der Formel (IV): 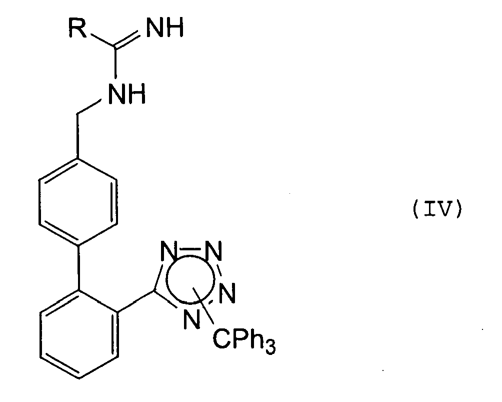
wobei R = ein C₂-C₇ geradkettiges oder verzweigtes Alkyl oder C₃-C₉-Cycloalkyl ist,
mit einer Verbindung ausgewählt aus 2-Brom-3-(1-methylethoxy)-2-propenal, 2-Chlormalonaldehyd oder 2-Brommalonaldehyd in Gegenwart einer Base in einem organischen Lösungsmittel, um eine Verbindung der Formel (III) zu bilden, wobei R wie oben definiert ist.

7. Verfahren gemäß Anspruch 6, **dadurch gekennzeichnet, daß** die Base ausgewählt ist aus Kaliumcarbonat und Triethylamin.

8. Verfahren gemäß Anspruch 6 oder 7, **dadurch gekennzeichnet, daß** das Lösungsmittel Dimethylformamid, Dimethylsulfoxid oder ein Alkohol ist, ausgewählt aus Methanol, Ethanol, n-Propanol, Isopropanol, n-Butanol oder Isobutanol.

9. Verfahren gemäß Anspruch 7, **dadurch gekennzeichnet, daß** das Lösungsmittel Dimethylformamid ist.

10. Verfahren gemäß Anspruch 6, ferner umfassend den Schritt:
Umwandeln einer Verbindung der Formel (V): 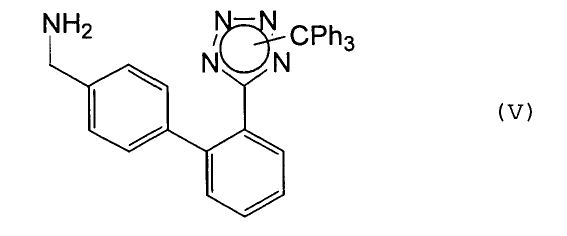 in ein Amidin der Formel (IV).

11. Verfahren gemäß einem der vorhergehenden Ansprüche,
wobei der Reduktionsschritt nach dem Chlorierungsschritt durchgeführt wird.

12. Verfahren gemäß einem der vorhergehenden Ansprüche,
wobei der Reduktionsschritt vor dem Chlorierungsschritt durchgeführt wird.

13. Verfahren zur Herstellung von 2-n-Butyl-1-[(2'-(2- oder 3-triphenylmethyl-2H-tetrazol-5-yl)-1,1'-biphenyl-4-yl)methyl]-1H-imidazol-5-carbaldehyd der Formel (IIIa): 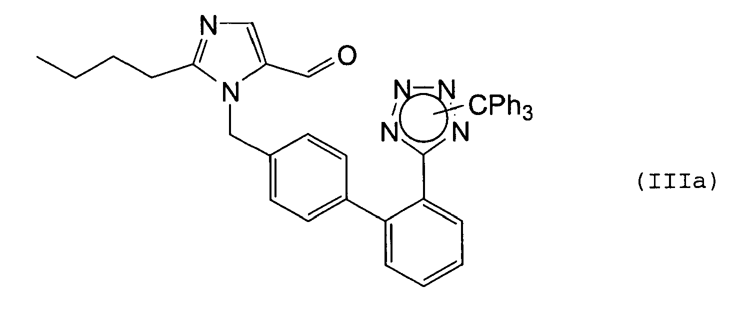 umfassend Umsetzen eines N-substituierten Amidins der Formel (IVa): 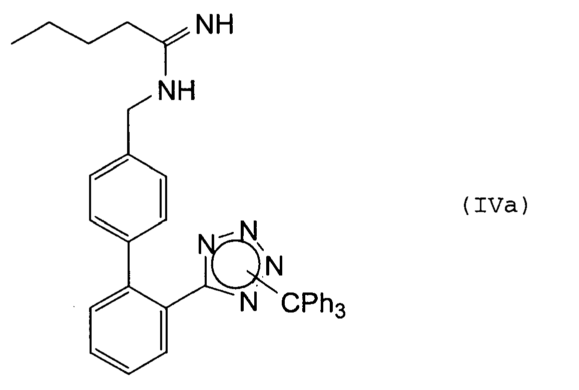 mit einer Verbindung ausgewählt aus 2-Brom-3-(1-methylethoxy)-2-propenal, 2-Chlormalonaldehyd oder 2-Brommalonaldehyd in Gegenwart einer Base in einem organischen Lösungsmittel.

14. Verbindung der Formel (IV) oder (IV)': 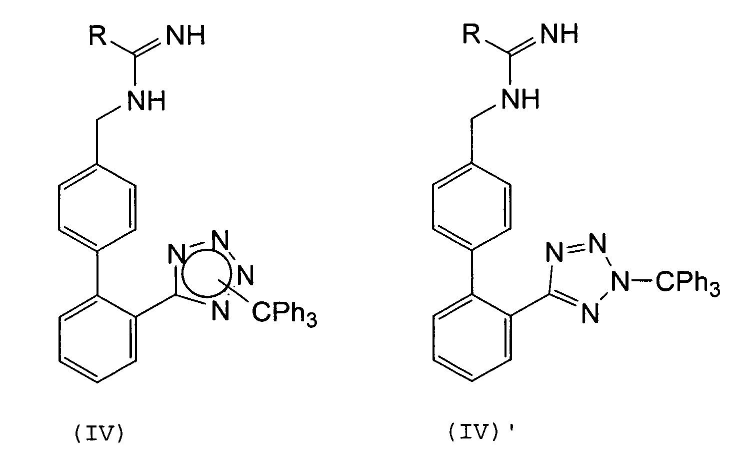 wobei R wie oben definiert ist.

15. Verwendung einer Verbindung gemäß Anspruch 14 als ein Intermediat in der Herstellung eines Sartanderivats oder ein pharmazeutisch akzeptables Salz davon, bevorzugt Losartan oder dessen Kaliumsalz.

## Revendications

1. Procédé de préparation d'un dérivé de sartan de formule (I) : 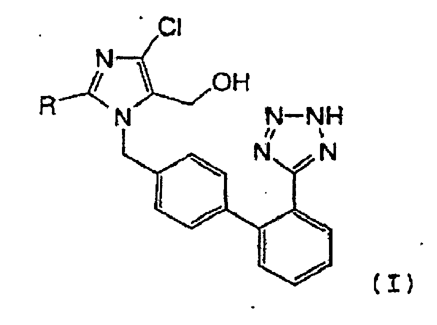 dans laquelle R = un alkyle à chaîne linéaire ou ramifiée en C₂-C₇ ou un cycloalkyle en C₃-C₉,
ou d'un sel pharmaceutiquement acceptable de celui-ci, comprenant les étapes de :
chloration et réduction, dans un ordre quelconque, d'un composé de formule (III) : 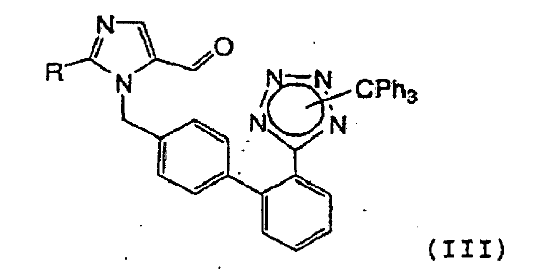
dans laquelle R est défini comme ci-dessus pour former un composé de formule (VI) : 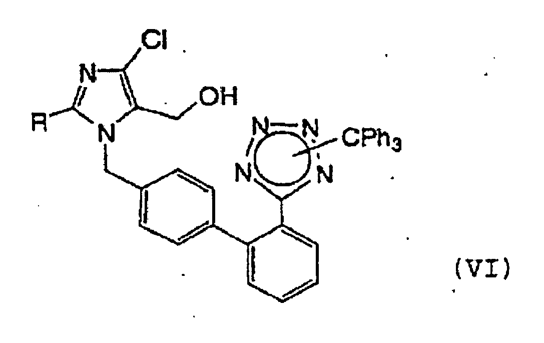 dans laquelle R est défini comme ci-dessus
et ensuite déprotection dudit composé de formule (VI) pour obtenir le dérivé de sartan de formule (I) et, facultativement, conversion dudit dérivé de sartan en l'un de ses sels pharmaceutiquement acceptables.

2. Procédé selon la revendication 1 dans lequel ledit dérivé de sartan est le losartan.

3. Procédé selon les revendications 1 et 2, dans lequel ledit sel pharmaceutiquement acceptable est le losartan potassium.

4. Procédé selon la revendication 1 **caractérisé en ce que** le réactif de chloration utilisé dans l'étape de chloration est choisi parmi un N-chloroimide, la 1,3-dichloro-5,5-diméthylhydantoïne, la 1,3-dichlorohydantoïne, NaOCl, BuOCl, ou t-BuOCl.

5. Procédé selon la revendication 4 **caractérisé en ce que** la chloration est effectuée dans le THF ou le diméthylacétamide.

6. Procédé selon l'une quelconque des revendications 1 à 5, comprenant en outre l'étape de :
réaction d'une amidine N-substituée de formule (IV) : 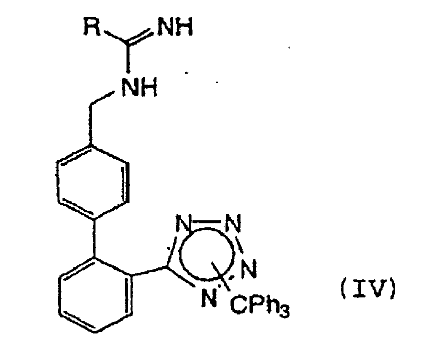
dans laquelle R = un alkyle à chaîne linéaire ou ramifiée en C₂-C₇ ou un cycloalkyle en C₃-C₉,
avec un composé choisi parmi le 2-bromo-3-(1-méthylétoxy)-2-propénal, le 2-chloromalonaldéhyde ou le 2-bromomalonaldéhyde en présence d'une base dans un solvant organique pour former un composé de formule (III) dans laquelle R est défini comme ci-dessus.

7. Procédé selon la revendication 6 **caractérisé en ce que** la base est choisie parmi le carbonate de potassium et la triéthylamine.

8. Procédé selon la revendication 6 ou 7 **caractérisé en ce que** le solvant est le diméthylformamide, le diméthylsulfoxyde ou un alcool choisi parmi le méthanol, l'éthanol, le n-propanol, l'isopropanol, le n-butanol ou l'isobutanol.

9. Procédé selon la revendication 7 **caractérisé en ce que** le solvant est le diméthylformamide.

10. Procédé selon la revendication 6 comprenant en outre l'étape de :
conversion d'un composé de formule (V) : 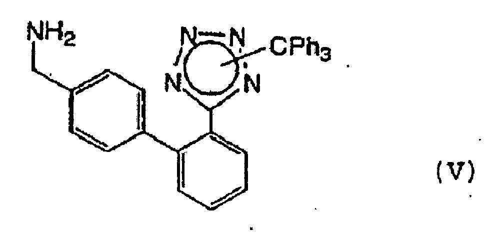
en une amidine de formule (IV).

11. Procédé selon l'une quelconque des revendications précédentes, dans lequel l'étape de réduction est exécutée après l'étape de chloration.

12. Procédé selon l'une quelconque des revendications précédentes, dans lequel l'étape de réduction est exécutée avant l'étape de chloration.

13. Procédé de préparation de 2-n-butyl-1-[(2'-(2- ou 3-triphénylméthyl-2H-tétrazol-5-yl)-1,1'-biphényl-4-yl)méthyl]-1*H*-imidazol-5-carbaldéhyde de formule (IIIa) : 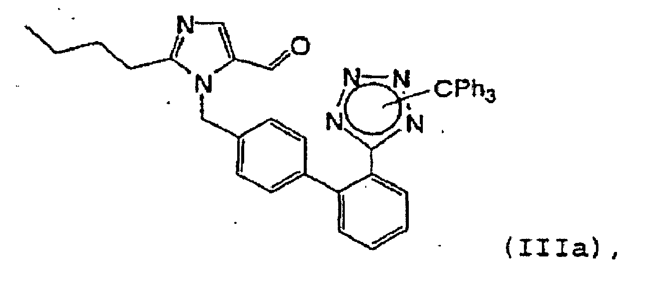 comprenant la réaction d'une amidine N-substituée de formule (IVa) : 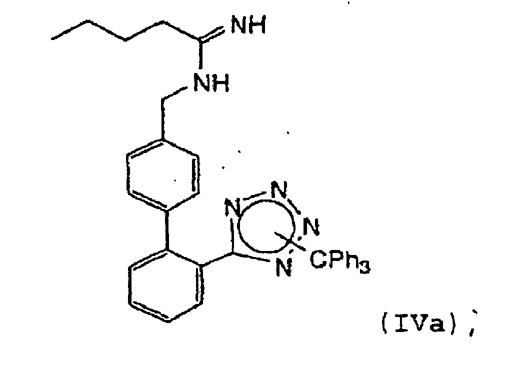 avec un composé choisi parmi le 2-bromo-3-(1-méthylétoxy)-2-propénal, le 2-chloromalonaldéhyde ou le 2-bromomalonaldéhyde en présence d'une base dans un solvant organique.

14. Composé de formule (IV) ou (IV)' : 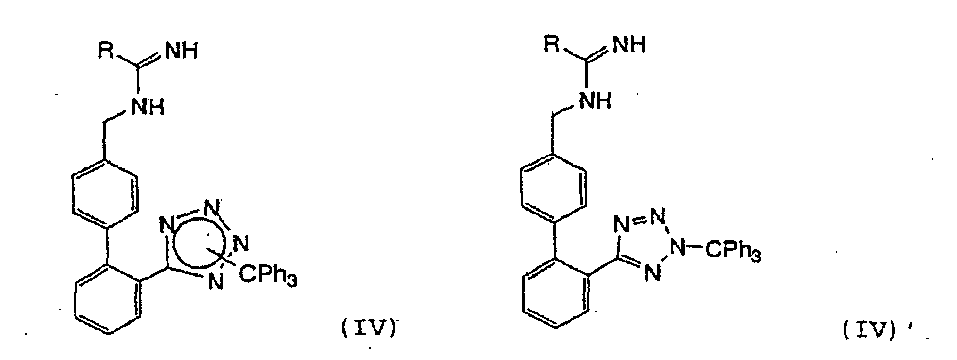 dans laquelle R est défini comme ci-dessus.

15. Utilisation d'un composé selon la revendication 14 comme un intermédiaire dans la préparation d'un dérivé de sartan, ou d'un sel pharmaceutiquement acceptable de celui-ci, préférablement de losartan ou de son sel de potassium.
